(19)

Europäisches
Patentamt
European
Patent Office
Office européen
des brevets

(11) **EP 3 989 808 B1**

(12) **EUROPEAN PATENT SPECIFICATION**

(45) Date of publication and mention
of the grant of the patent:
**05.11.2025 Bulletin 2025/45**

(21) Application number: **20832624.9**

(22) Date of filing: **26.06.2020**

(51) International Patent Classification (IPC):
***A61B 5/02*** *(2006.01)*

(52) Cooperative Patent Classification (CPC):
**A61B 5/02116; A61B 5/02416; A61B 5/7221**

(86) International application number:
**PCT/US2020/040005**

(87) International publication number:
**WO 2020/264439 (30.12.2020 Gazette 2020/53)**

(54) **APPARATUS, SYSTEMS, AND METHODS FOR NONINVASIVE MEASUREMENT OF CARDIOVASCULAR PARAMETERS**

VORRICHTUNG, SYSTEME UND VERFAHREN ZUR NICHTINVASIVEN MESSUNG KARDIOVASKULÄRER PARAMETER

APPAREIL, SYSTÈMES ET PROCÉDÉS DE MESURE NON INVASIVE DE PARAMÈTRES CARDIOVASCULAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.06.2019 US 201962867784 P**

(43) Date of publication of application:
**04.05.2022 Bulletin 2022/18**

(73) Proprietor: **Vital Metrix, Inc.
Huntsville AL 35806 (US)**

(72) Inventors:
• **BAYYUK, Sami
Huntsville, AL 35802 (US)**

• **HEYM, Jason
Huntsville, AL 35801 (US)**
• **REICH, Alton
Huntsville, AL 35806 (US)**

(74) Representative: **Invent Horizon IP
Nordenskiöldsgatan 11A
211 19 Malmö (SE)**

(56) References cited:
| | |
|---|---|
| **WO-A1-2017/220353** | **CN-B- 103 020 472** |
| **US-A1- 2014 073 878** | **US-A1- 2014 081 100** |
| **US-A1- 2014 276 115** | **US-A1- 2016 128 582** |
| **US-A1- 2016 302 679** | **US-A1- 2018 344 219** |

EP 3 989 808 B1

**Description**

BACKGROUND OF THE INVENTION:

Field of the Invention

The invention relates to medical apparatus, methods, systems, and non-transitory computer-readable storage media for non-invasive measurement of physiological parameters of the cardiovascular system using plethysmographic pulse wave or pulse pressure wave data.

Description of Related Art

WO 2010/124034, US 8,494829 B1, US 9,060,722 B1, US 9,173,574 B1, US 9,375,171, US 9,451,886 B1, US 9,649,036 B1 US 2017/0119261 A1, and EP 2512325 B1 disclose non-invasive systems and methods for estimating or measuring cardiovascular parameters using data from a pulse oximeter. While these are able to estimate or measure cardiovascular parameters noninvasively, the computing power involved is considerable and requires the use of powerful computers not found in laptops, PCs, tablets, or other computing devices commonly found in medical offices or homes. Consequently, there remains a need for non-invasive systems, apparatus, and methods that are capable of measuring cardiovascular parameters such as cardiac output, stroke volume, aortic pressure, and venous pressure in real time or within several minutes using the types of processors and computers commonly present in computing devices found in medical offices and homes.

WO2017/220353 discloses a method to analyze and classify cardiovascular waveforms in a manner that better identifies cardiovascular abnormalities and corroborates or refutes classifications made using existing algorithms using a template-based analysis and classification of cardiovascular waveforms. The templates can include normal and abnormal waveforms to classify periodic components of hemodynamic waveforms. Analyzing may include matching the corresponding periodic component of a hemodynamic waveform to a template of the database of hemodynamic templates.

BRIEF SUMMARY OF THE INVENTION:

The present invention provides a computer implemented method for noninvasively measuring a value of cardiovascular parameter of a subject as defined in claim 1, a system for monitoring a cardiovascular parameter as defined in claim 13 and a non-transitory computer-readable storage medium as defined in claim 15. Preferred embodiments of the invention are defined in the dependent claims.

The present invention fills a need in the art for biomedical monitoring devices capable of noninvasively measuring cardiovascular parameters such as cardiac output (CO), stroke volume (SV), venous pressure (VP), aortic pressure (AP), and arterial or venous resistance, compliance, inertance, and regurgitation fraction. Technical features including a computer generated, or synthetic, library of pulse plethysmographic waveforms or pulse pressure waveforms (PWs) and computer implemented methods for processing plethysmographic or pulse pressure input data and screening the synthetic library to contribute to solving the problems of reducing required processing time and improving accuracy compared to earlier apparatus and methods. A computer implemented algorithm comprising a data collection module, a data qualifying module, and a screening module accepts plethysmographic or pulse pressure data comprising PWs, qualifies a subset of PWs for screening, and screens the subset of PWs against a synthetic PW library to establish which synthetic PW is the closest fit to the qualified PW subset. One or more cardiovascular parameters associated with the PW having the closest fit may be reported as the measured cardiovascular parameter. The computer implemented algorithm is capable of providing a measured cardiovascular parameter such as CO or SV within minutes or in real time from PW data provided by a pulse oximeter or other plethysmographic measuring device or a device capable of measuring pulse pressure waveforms. An output module for displaying and/or transmitting output data may optionally be coupled to a lookup table comprising data related to a drug and/ or medical device to produce an output that includes an instruction, a notation, and/or a recommendation for a drug dosage, a drug administration, and/or a change in, or an initiation of, an operation of a medical device.

BRIEF DESCRIPTION OF THE DRAWINGS:

**FIG. 1** is a flowchart illustrating steps in an exemplary embodiment of a method;

**FIGs. 2A-C** are graphs of a series of pulse plethysmographic or pulse pressure waveforms (PWs) including indications of cycle boundaries;

**FIG. 3** is a flow chart showing an example of how quality metrics are applied to a series of PWs;

**FIGs. 4A and B** show a PW before and after normalization;

**FIG. 5** illustrates a screening of a synthetic PW library;

**FIG. 6** is a graph of computer generated PWs;

**FIG. 7** is a graph depicting examples of computer generated PWs; and

**FIG. 8** is a chart of one embodiment of software modules for executing algorithms.

DETAILED DESCRIPTION OF THE INVENTION:

All art specific terms used herein are intended to have their art-accepted meanings in the context of the description unless otherwise indicated. All non-art specific terms are intended to have their plain language meaning in the context of the description unless otherwise indicated.

As used herein, the term "PW' is an abbreviation for "plethysmographic waveform," "pulse plethysmographic waveform" or "pulse pressure waveform," which may also be expressed as "plethysmographic or pulse pressure waveform." The term "plethysmography" relates to measuring changes over time in blood volume. Time varying pulse pressure may be measured directly or indirectly from plethysmography data. Plethysmography waveforms may comprise measured pulse volume vs. time or fraction of heart cycle. Plethysmography waveforms may comprise pulse pressure as measured by plethysmography vs. time or fraction of heart cycle. Pulse pressure waveforms may be measured by means other than plethysmography.

The invention involves the use of a plethysmographic or pulse pressure waveform (PW) library comprising a large number of simulated PWs that may be generated using a computational fluid dynamic model of a cardiovascular system. For human subjects, the model is of a human cardiovascular system. The invention applies to nonhuman subjects as well, so long as the cardiovascular system is understood well enough to model and plethysmography or pulse pressure PW data may be collected. The human cardiovascular system is used for the purpose of describing the invention.

The most commonly used plethysmography methods and apparatus are pulse oximetry (PO) devices that use absorption of light at two wavelengths corresponding to the absorption maxima for oxygenated and deoxygenated forms of hemoglobin. Changes in absorption are related to changes in blood volume and changes in pressure can be derived from changes in blood volume. Time varying blood volume may additionally or alternatively be measured using ultrasound to measure the diameter of blood vessels. Time varying pulse pressure waveforms may also be measured directly using a partially inflated blood pressure cuff, pressure transducers, strain gages or stretch sensors. The systems, apparatus, and methods are described herein using pressure as a parameter that changes with time. This is convenient because PO data is commonly converted to pressure vs. time data.

Pressure need not be used as the time varying parameter of plethysmographic data. Instead, PW cycles may be in the form of volume vs. time or absorption vs. time, for example. PO devices use two wavelengths so that amounts of oxygenated and deoxygenated hemoglobin may be compared to report a percent oxygenation of hemoglobin. While the usual PO data is used for the examples herein, the apparatus and methods herein may use data collected using, for example, only changes in absorption by oxygenated hemoglobin or changes in blood volume or blood pressure derived therefrom. PO data is most often collected at fingertips but may additionally or optionally be measured on other extremities such as ears or toes and reflectance or scattering PO measurements may be made at locations on the body that are not compatible with transmission PO measurements.

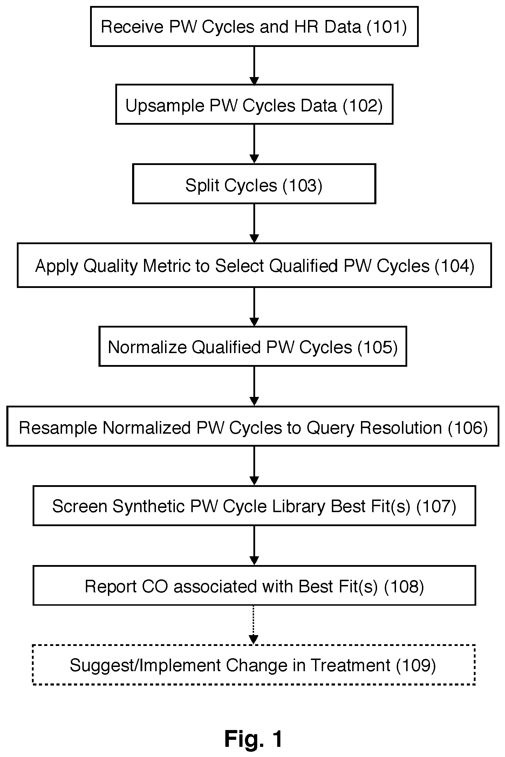

**Fig. 1** is a flowchart showing processes that may be performed in a method for measuring a cardiovascular parameter using plethysmographic PW or pressure PW data. Plethysmography PW data, from a measuring device (801) such as a pulse oximeter, is received (101) into a data collection module of the computer implemented method software. For the embodiments shown in the drawings, the PW data comprises pressure or volume or light transmission or absorption vs. time data. A heart rate (HR) associated with the PW data is also received (101) into the data collection module. Data may be collected from a real time measurement and/or stored data.

In the case of PO data, the pressure value is determined using a photoplethysmographic calculation based upon the absorption of oxyhemoglobin and/or deoxyhemoglobin over time. Other types of plethysmography may measure pressure or volume directly, for example via stretch sensors or sphygmomanometry. In other embodiments, the PW data may comprise volume vs. time data or absorbance vs. time data, for example. A cardiovascular system model may include blood light absorbance, blood pressure, and/or blood volume as parameters so that any of these parameters alone, or in any combination may be used in place of pressure vs. time.

After the data collection module (801) has received (101) PW data, the cycles are divided into a series of discrete cycles in a cycle splitting (103) process. A quality metric may be applied (104) to cycles and/or runs of individual cycles to select one or more PW cycles for screening against a synthetic PW library (807). Selected, or qualified, PW cycles are preferably normalized (105) before screening (107) to produce PW cycles in the form of pressure vs. fraction of cycle. This allows a simpler and faster screening of the PW library (807). Comparing a library of normalized synthetic PWs, each with an associated HR, to normalized qualified PWs in the screening step (107) reduces the number of synthetic PWs required because PW curves having different HRs can have the same shape when normalized.

A computational screening process (107) in which the synthetic PW library is screened to identify the synthetic PW(s) most similar in shape to qualified PW(s) produces a final output of a SV/CO and/or other cardiovascular parameters corresponding to the synthetic PW that best matches the qualified PW(s) or combination of synthetic PWs that best match the qualified PW(s). The output is reported (108), for example by display on a monitor and/or storage in an accessible database for recall.

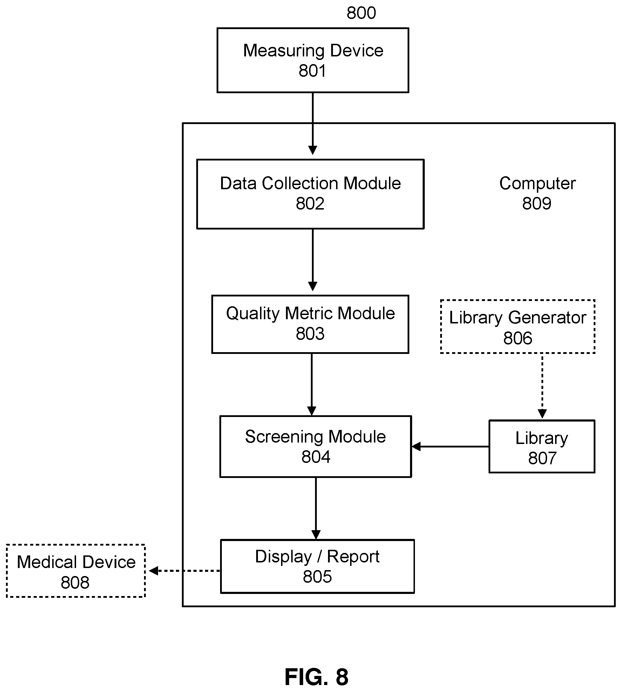

An optional addition to the reporting (108) process may include the generation of a recommendation or suggestion (109) for a change in medication or dose of medication to affect a cardiovascular parameter being reported (**Fig. 8**). Examples of medication-related recommendations include a recommendation to increase a dosage, a recommendation to decrease a dosage, and a recommendation to switch to a different medication. Additionally or alternatively, a reporting process may include a recommendation and/or instruction for changing or controlling an operational parameter of a medical device configured to alter or regulate a cardiovascular parameter being reported. Examples of a medical device configured to alter or regulate a cardiovascular parameter include a heart assist device and a pump that increases CO. Examples of controlling an operational parameter of a medical device include adjusting the speed of a heart pump to increase or decrease the flow rate and triggering a medication dosing pump to provide a dose or modified dose of medication. Additionally or alternatively, the reporting process (108) may include a recommendation and/or instruction (109) for changing a person's diet or exercise routine. Examples of a recommendation for changing a person's diet or exercise routine may include a recommendation to increase or decrease fluid intake, a recommendation to increase or decrease salt intake, a recommendation to increase or decrease a physical activity, and a recommendation to consult a health care professional.

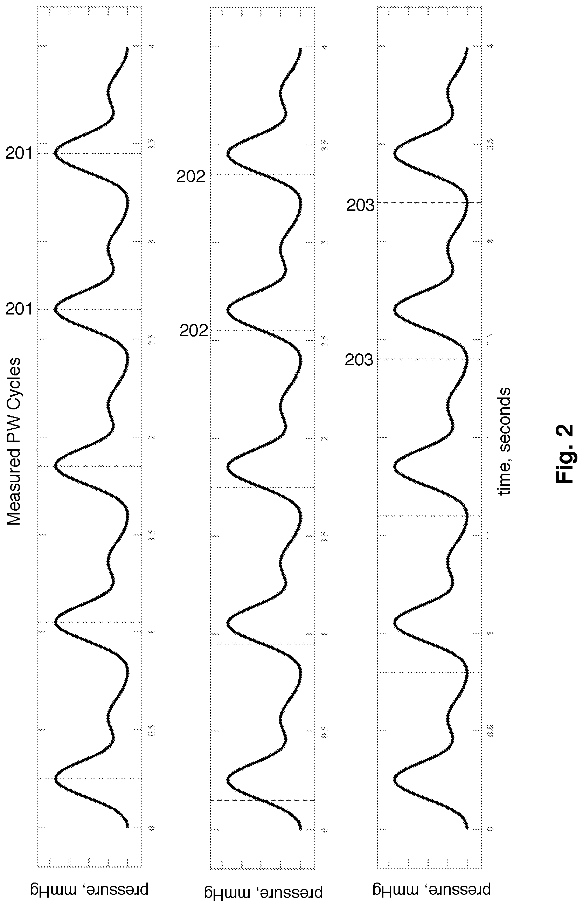

The PW data received comprises a series PW cycles with each cycle corresponding to a complete heart cycle. The method involves splitting the continuous series of cycles (103) into discrete, identifiable cycles, which may be defined from any point in a cycle to the same point in the following cycle (**Figs. 2A-C**). For example the cycle may be measured from the beginning of diastole (201) in a heart cycle to the beginning of diastole (201) in the next (**Fig. 2A**), from the middle of systole (202) in a heart cycle to the middle of systole (202) in the next (**Fig. 2B**) from the beginning of systole (203) in a heart cycle to the beginning of systole (203) in the next (**Fig. 2C**), and so forth. A series of PW cycles may comprise, for example, from 0.1 minutes to 60 minutes of continuous or discontinuous PW data from a subject.

A pulse oximeter may have a sampling rate of from 50 Hz to 500 Hz. The identification of the beginning and end of each PW cycle may optionally involve upsampling (102) of the PW data to a higher resolution, particularly in the vicinity of the start and stop points of the cycles, to improve the accuracy of start and stop point determination. Upsampled frequencies may be from 60 Hz to 5,000 Hz, with higher resolution requiring more time and generally providing higher accuracy within limits than lower frequencies. Upsampling may be applied uniformly within cycles or non-uniformly.

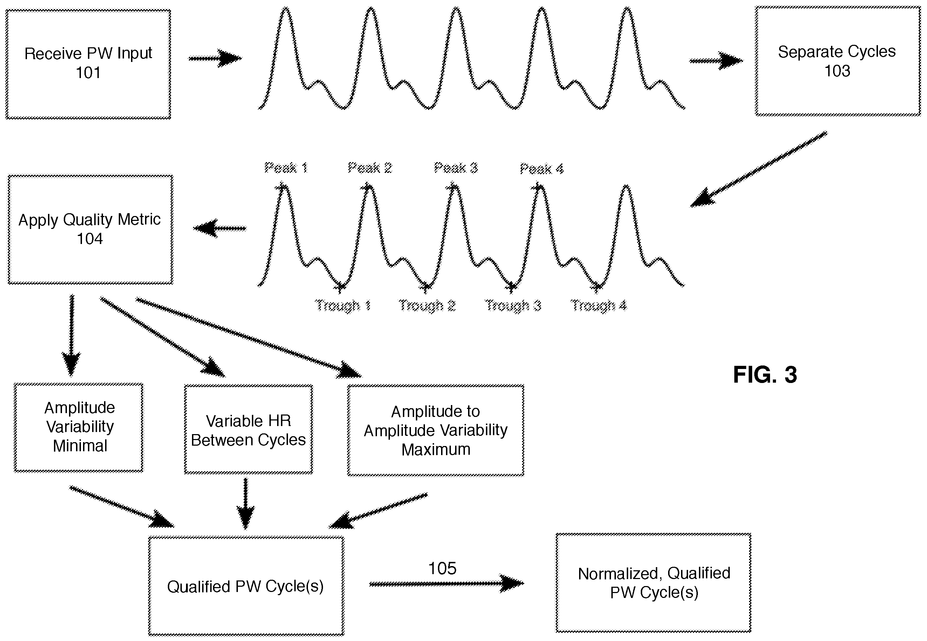

HR, SV, CO, and other cardiovascular parameters are not constant over time and may change from beat to beat. Any measurement of this type of parameter, therefore, is necessarily an averaged value. The constant variation in measured pulse pressures, duration of cycles, and shapes of PWs, are complicating factors for the measurement of SV, CO, and other cardiovascular parameters. Additionally, movement of the subject and other physical disturbances cause artifacts in the data. A quality metric is applied (104) to subject PW data by a quality metric module (803) to minimize the effects of artifacts and outliers on the subsequent screening processes. Different quality metrics and combinations of metrics may be used to select qualified PWs for comparison to, or screening against (107), a synthetic PW library (807). For example, an algorithm may select a set of individual cycles and/or runs of cycles that have, in combination, a high ratio of cycle amplitude to cycle amplitude variability, minimum localized plethysmographic or pressure wave amplitude variability, and minimum HR variability (Fig. 3). A high ratio of amplitude to amplitude variability is indicative of a clear measurement containing minimal noise. High localized plethysmographic or pressure wave amplitude variability is indicative of sensor movement and blood pressure cuff constriction artifacts. Quality metrics involving amplitude require that the peaks and troughs of PW cycles be identified, whether these are used as cycle break points or not. Minimization of HR variability in qualified PWs tends to improve the accuracy of estimated SV and CO measurement output. The total number of qualified PW cycles selected may be from 1 to 25, preferably from 5 to 25 cycles. The number may be higher than 25 but may not improve accuracy while increasing processing time.

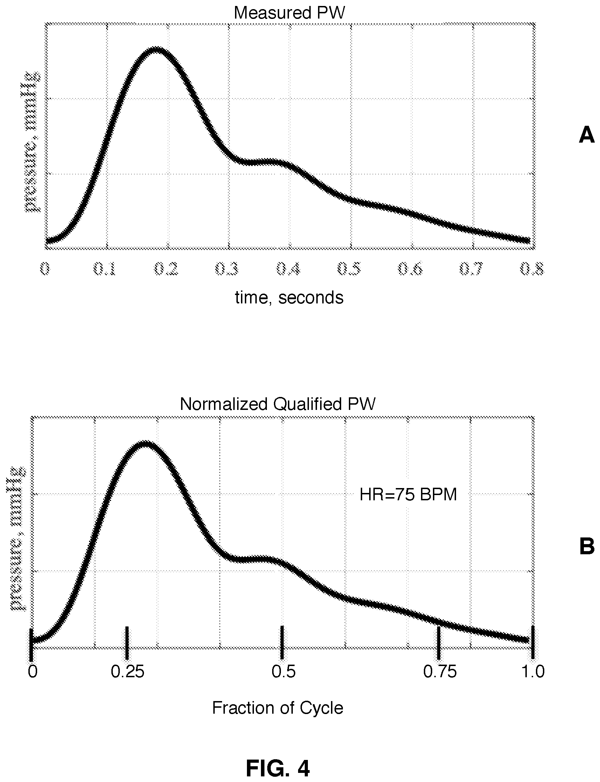

Before the qualified PW cycles are compared (107) to the synthetic PW library (807), the qualified PW cycles are preferably normalized (105) and phased with the synthetic PWs by a quality module (803) or a screening module (804) (**Fig. 8**). Each synthetic PW comprises pressure vs. fraction of cycle data and an associated HR. The screening is based on a difference between two curves, the curve of each qualified PW as represented by a series of data points and each synthetic PW, also represented by a series of data points. To perform the screening, the qualified PW data points, which comprise pressure vs. time data, are normalized to pressure vs. fraction of cycle (**Fig. 4**). The normalized PW data and synthetic PWs from the library have the same resolution, i.e. points per cycle, and are in phase so that each point in each noralized PW has a point at a corresponding fraction of a cycle in each synthetic PW With this arrangement, a difference, or error, may be calculated for each comparison of a point on a qualified PW and its corresponding point in a synthetic PW.

Normalization (105) uses the HR associated with the measured PW to convert pressure vs. time to pressure vs. fraction of cycle and the HR value remains associated with the normalized PWs. Depending on the resolution of the qualified PWs and the resolution of the synthetic PWs, the qualified PWs may be upsampled or downsampled to match the resolution of the synthetic PWs, which is preferably between 75 Hz and 1200 Hz. Methods for resampling, upsampling (interpolation), and downsampling (decimation) and phase matching of sinusoidal waveform data are well known and are therefore not described in detail. The upsampling and/or downsampling may be not applied uniformly in time along each split cycle or non-uniformly.

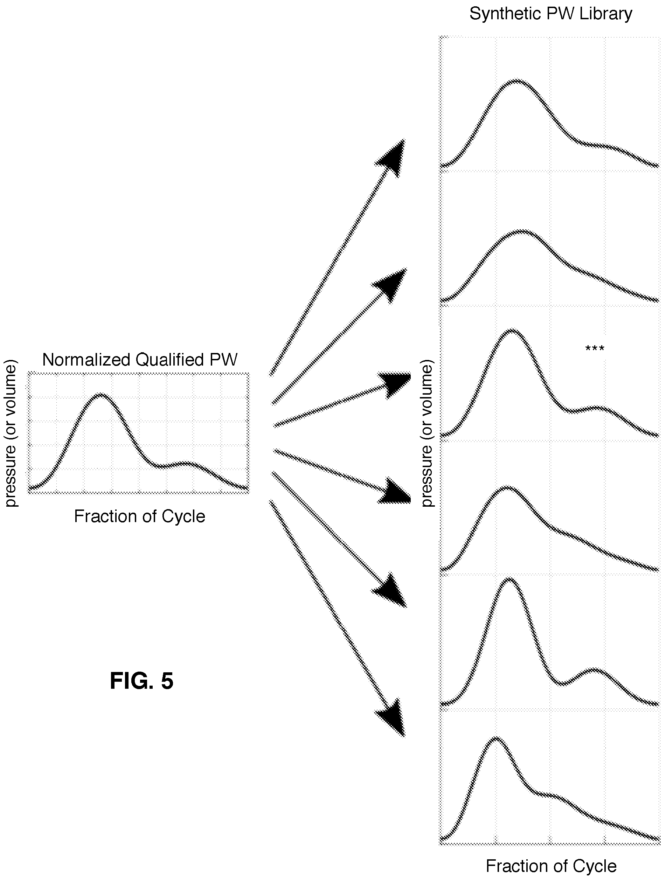

Screening (107) of the synthetic PW library (807) comprises a geometric comparison of points on normalized curves for one or more best match or best fit solutions. The normalized curves may have a resolution of, for example, from 75 Hz to 5,000 Hz. A best match, or best fit, solution may be defined as the synthetic PW(s) having the minimum summed error when compared to one or more query PW cycles, or cycles being used to screen the library. A screening module (804) comprises a computer program comprising an algorithm configured to determine best match solution(s). To illustrate the process, **Fig. 5** shows a normalized, qualified PW cycle and a series of synthetic PW cycles against which the qualified PW cycle is screened. It may be seen that the library PW marked with three asterisks is the closest match to the qualified PW cycle. In practice, the library contains such a large of library members, that it impossible to find the closest match by visual inspection. The screening module (804) comprises an algorithm that achieves the identification of the synthetic PW cycle(s) that best fit one or a plurality of qualified PW cycles.

The screening module (804) may comprise a program for executing a Kernel method algorithm. Kernel methods are a class of machine learning algorithms for pattern analysis, including the well known support vector machine (SVM). Kernel methods use kernel functions, which enable them to operate in a high-dimensional, implicit feature space without computing the coordinates of the data in the space. Instead, the method computes the inner products between the images of all pairs of data in the feature space. Algorithms capable of operating with kernels include SVMs, Gaussian processes, principal components analysis, canonical correlation analysis, ridge regression, spectral clustering, and linear adaptive filters. Any linear model can be turned into a non-linear model by replacing its features (predictors) by a kernel function.

The normalized, qualified PW cycles may be compared to synthetic PW cycle library members as individual qualified PW cycles or as a continuous series of qualified PW cycles. It is preferred that comparing qualified cycles to synthetic cycles is performed with the qualified cycles combined into a single, continuous series of cycles. This allows a more powerful consensus or voting style effect on estimated parameters to be achieved without explicitly calculating a consensus or vote for each cycle.

A corresponding multiplied-dimension distance or similarity function kernel may be used to operate on multi-cycle PW data in conjunction with the library. This achieves a consensus or voting style effect that allows beat-to-beat variability, such as HR variations, to reinforce other estimated parameters without imposing restrictions on the beat-to-beat variability. The use of locally-selected plethysmographic or pressure wave cycles to multiply the dimensionality of the distance or similarity kernel improves accuracy when compared to averaging estimated parameters for each query and adjusting the K factor in K nearest neighbor (KNN) queries. If more than one nearest neighbor is selected, for example the K factor equals 5, the 5 nearest neighbors may be reduced to a single solution by average, weighted average, etc. of parameter values of the nearest neighbors.

The computer generated library (807) of synthetic, PW cycles comprises at least millions of unique simulated, or synthetic, PW cycles with each PW cycle having a corresponding HR. The entire library need not be screened because the normalized PW(s) used to screen the library has (have) an associated HR that is sufficiently close to the nearest library HRs so that only library members having the same HR or the closest two or three HRs are included in the screening (107). Synthetic PW cycles and the qualified PW cycles may comprise additional parameters such as age, height, weight, gender, body mass index, and/or measurement site on the subject. Associating the same subject specific parameter(s) with the PW data may allow further reduction in the number of synthetic PW cycles to include in the query.

The synthetic PW cycle library (807) may be generated in many ways, for example using an empirical computational model, a computational fluid dynamics (CFD) model or electrical model analog of a fluid dynamics model, or any combination of these. Additionally or alternatively, a synthetic PW cycle library may be generated using measured data collected from living subjects and may be computationally normalized and/or supplemented with additional parameters such as age, height, weight, gender, body mass index, and/or measurement site on the subject. A model for generating the library should comprise model parameters corresponding in some way to cardiovascular parameters used as input and cardiovascular parameters to be measured. A computational model may use mathematical representations of physiological observations that correspond indirectly to one or more physiological processes, such as mathematical representations of signals obtained from sensor data or empirically fitting a mathematical equation to data collected from a physiological source. For example, a cardiovascular system model may be functionally coupled to or contain a computational model that simulates the signal obtained from a photoplethysmogram.

**Example I - Computational Fluid Dynamic (CFD) model of human cardiovascular system (HCS):** A computational model for generating a synthetic PW cycle library may comprise a CFD human cardiovascular system (HCS) model. Such a CFD HCS model may comprise CFD model segments corresponding to various physiological segments of the human cardiovascular system such as the heart, aorta, arteries, capillaries, veins, and pulmonary system. In a preferred embodiment, the CFD HCS model comprises a closed-loop chain of one-dimensional hydrodynamic elements with each element corresponding to a segment of anatomical vascular structure(s) and represented by an elastic channel having its own resistance, compliance, and inertance. An element representing the heart acts as a positive displacement pump.

In one example, each element of the model may be governed by a set of equations:

$$\frac{dV_{e_i}}{dt} = \dot{Q}_{n_i} - \dot{Q}_{n_{i+1}} \tag{1}$$

$$V_{e_i} = C_{e_i}\left(P_{e_i} - P_{e_i}^0\right) + V_{e_i}^0 \tag{2}$$

$$P_{n_i} - P_{e_i} = \frac{R_i}{2}\dot{Q}_{n_i} + \frac{l_i}{2}\frac{d}{dt}\left(\dot{Q}_{n_i}\right) \tag{3}$$

$$P_{e_i} - P_{n_{i+1}} = \frac{R_i}{2}\dot{Q}_{n_{i+1}} + \frac{l_i}{2}\frac{d}{dt}\left(\dot{Q}_{n_{i+1}}\right) \tag{4}$$

where $Ve_i$ is the volume of element $i$, $\dot{Q}_{ni}$ is the volume flow rate at node $i$, $Ce_i$ is the time-averaged and length-averaged hydrodynamic compliance of element $i$, $Pn_i$ is the pressure at node $i$, $Pe_i$ is the pressure at the center of element $i$, $Ri$ is the time-averaged and length-averaged hydrodynamic resistance of element $i$, and $li$ is the length of element $i$.

In this example, equation (1) is a discrete form of the volume conservation equation with blood being treated as an incompressible fluid and volume conservation being equivalent to mass conservation. Equation (2) is a discrete form of a constitutive relation for the compliance of an element. Equations (3) and (4) are discrete forms of momentum conservation equations for, respectively, the left-hand half and the righthand half of the element. Equations (1)-(4) are four independent equations relating the six variables $Pei$, $Vei$, $Pni$, $Qni$, $Pni+1$, and $Qni+1$ of each element and its two nodes. For the system as a whole, the number of unknowns is

$$2Ne + 2Nn = 2Ne + 2(Ne + 1) = 4Ne + 2 \tag{5}$$

while the close-circuit condition provides the two constraints

$$PnNnodes = Pn1 \tag{6}$$

and

$$\dot{Q}nNnodes = \dot{Q}n1 \tag{7}$$

The total number of unknowns with the closed-circuit condition is given by

$$(4Ne + 2) - 2 = 4Ne \tag{8}$$

Since the total number of independent unknowns equals the total number of independent equations, the system of governing equations has a unique solution.

**Example II - Windkessel model for HCS Model:** A computational model for generating a synthetic PW cycle library may comprise a Windkessel model representing parts or all of the HCS. The following is an example of a suitable Windkessel comprising model for generating a synthetic PW library. In this example, particular equations are used to describe a dynamic state-space model.

Cardiac output is represented by equation 9,

$$Q_{CO}(t) = \overline{Q}_{CO} \sum_{1}^{8} a_k \exp\left[\frac{-(t - b_k)^2}{c_k^2}\right] \tag{9}$$

where cardiac output $Q_{co}(t)$, is expressed as a function of heart rate (HR) and stroke volume (SV) and where $Q_{co} = (HR \times SV)/60$. The values $a_k$, $b_k$, and $c_k$ are adjusted to fit data on human cardiac output.

The cardiac output function pumps blood into a Windkessel 3-element model of the vascular system including two state variables: aortic pressure, $P_{ao}$, and radial (Windkessel) pressure, $P_w$, according to equations 10 and 11,

$$P_{w,k+1} = \frac{1}{C_w R_p}\left(\left(R_p + Z_0\right)Q_{CO} - P_{CO,k}\right)\delta t + P_{w,k} \tag{10}$$

$$P_{ao,k+1} = P_{w,k+1} + Z_0 Q_{CO} \tag{11}$$

where $R_p$ and $Z_o$ are the peripheral resistance and characteristic aortic impedance, respectively. The sum of these two terms is the total peripheral resistance due to viscous (Poiseuille-like) dissipation according to equation 12,

$$Z_0 = \sqrt{\rho / AC_l} \tag{12}$$

where $\rho$ is blood density and $C_l$ is the compliance per unit length of artery. The elastic component due to vessel compliance is a nonlinear function including thoracic aortic cross-sectional area, A: according to equation 13,

$$A(P_{CO}) = A_{max}\left[\frac{1}{2} + \frac{1}{\pi}\arctan\left(\frac{P_{CO} - P_0}{P_1}\right)\right] \tag{13}$$

where $A_{max}$, $P_0$, and $P_1$ are fitting constants correlated with age and gender that may be of a form similar to equations 14-16.

$$A_{max} = \left(5.62 - 1.5(gender)\right) \cdot cm^2 \tag{14}$$

$$P_0 = \left(76 - 4(gender) - 0.89(age)\right) \cdot mmHg \tag{15}$$

$$P_1\left(57 - 0.44(age)\right) \cdot mmHg \tag{16}$$

The time-varying Windkessel compliance, $C_w$, and the aortic compliance per unit length, $C_l$, are related in equation 17,

$$C_w = lC_l = l\frac{dA}{dP_\infty} = l\frac{A_{max}/(\pi P_1)}{1 + \left(\frac{P_\infty - P_0}{P_1}\right)} \tag{17}$$

where $l$ is the aortic effective length. The peripheral resistance is defined as the ratio of average pressure to average flow. A set-point pressure, $P_{set}$, and the instantaneous flow related to the peripheral resistance, $R_p$, according to equation 18,

$$R_P = \frac{P_{set}}{(HR \cdot SV)/60}$$

(18)

are used to provide compensation to autonomic nervous system responses. The value for $P_{set}$ is optionally adjusted manually to obtain 120 over 75 mmHg for a healthy individual at rest.

The compliance of blood vessels changes the interactions between light and tissues with pulse. This is accounted for using a homogenous photon diffusion theory for a reflectance or transmittance pulse oximeter configuration according to equation 19,

$$R = \frac{I_{ac}}{I_{dc}} = \frac{\Delta I}{I} = \frac{3}{2} \sum_{s}^{1} K(\alpha, d, r) \sum_{a}^{art} \Delta V_0$$

(19)

for each wavelength. In this example, the red and infrared bands are centered at about $660 \pm 100$ nm and at about $880 \pm 100$ nm. In equation 19, $I$ (no subscript) denotes the detected intensity, $R$, is the reflected light, and the alternating current intensity, $I_{ac}$, is the pulsating signal, ac intensity, or signal; and the background intensity, $I_{dc}$, is the direct current intensity or dc intensity; $\alpha$, is the attenuation coefficient; $d$, is the illumination length scale or depth of photon penetration into the skin; and $r$ is the distance between the source and detector. $V_a$ is the arterial blood volume, which changes as the cross-sectional area of illuminated blood vessels, $\Delta A_w$, according to equation 20,

$$\Delta V_a \approx r \cdot \Delta A_w$$

(20)

where r is the source-detector distance.

The tissue scattering coefficient, $\sum_{s}'$, is assumed constant but the arterial absorption coefficient, $\sum_{a}^{art}$, which represents the extinction coefficients, depends on blood oxygen saturation, $SpO_2$, according to equation 21,

$$\sum_{a}^{art} = \frac{H}{v_i} \left[ SpO_2 \cdot \sigma_0^{100\%} + (1 - SpO_2) \cdot \sigma_0^{0\%} \right]$$

(21)

which is the Beer-Lambert absorption coefficient, with hematocrit, $H$, and red blood cell volume, $v_i$. The optical absorption cross-sections, proportional to the absorption coefficients, for red blood cells containing totally oxygenated ($HbO_2$) and totally deoxygenated (Hb) hemoglobin are $\sigma_a^{100\%}$ and $\sigma_a^{0\%}$, respectively.

The function $K(\alpha, d, r)$, along with the scattering coefficient, the wavelength, sensor geometry, and oxygen saturation dependencies, alters the effective optical path lengths, according to equation 22.

$$K(\alpha, d, r) \approx \frac{-r^2}{1 + \alpha r}$$

(22)

The attenuation coefficient $\alpha$ is provided by equation 23,

$$\alpha = \sqrt{3 \sum_a \left( \sum_s + \sum_a \right)}$$

(23)

where $\Sigma_a$ and $\Sigma_s$ are whole-tissue absorption and scattering coefficients, respectively, which are calculated from Mie Theory.

Red, $\overline{K_r}$, and infrared, , $\overline{K_{ir}}$, K values as a function of $SpO_2$ are optionally represented by two linear fits, provided in equations 24 and 25

$$\overline{K_r} \approx -4.03 \cdot SpO_2 - 1.17 \tag{24}$$

$$\overline{K_{ir}} \approx 0.102 \cdot SpO_2 - 0.753 \tag{25}$$

in $mm^2$. The overbar denotes the linear fit of the original function. The pulsatile behavior of $\Delta A_w$, which couples optical detection with the cardiovascular system model, is provided by equation 26,

$$\Delta A_w = \frac{A_{w,max}}{\pi} \frac{P_{w,1}}{P_{w,1}^2 + \left(P_{w,k+1} - P_{w,0}\right)^2} \Delta P_w \tag{26}$$

where $P_{w,0}=(1/3)P_0$ and $P_{w,1}=(1/3)P_1$ account for the poorer compliance of arterioles and capillaries relative to the thoracic aorta. The subscript $k$ is a data index and the subscript $k+1$ or $k+n$ refers to the next or future data point, respectively.

Each PW cycle corresponds to a complete heart cycle, or heart beat, and is associated with its corresponding HR. Because the system is closed, the flow rate (volume/time) through each segment is the same when the system has reached a steady state, giving each solution a corresponding CO. Because HR and CO are known, SV is also known. The pressure within each segment is also known. Consequently, if a segment corresponds to the aorta the aortic pressure may be the cardiovascular parameter measured. If a segment corresponds to the veins, central venous pressure may be the cardiovascular parameter measured. Other HCS segments may include the arteries, arterioles, capillaries, venules, veins, vena cava, pulmonary artery, and the complete pulmonary system, for example.

The HCS CFD model is initiated with a set of initial conditions, such as segment volumes and pressures, and nodal flow rates. The model is then solved iteratively to evolve the volumes, pressures, and flow rates forward in time. The change in model parameters, such as the volume of an element, from one simulated cycle (beat) to the next is used to judge the convergence of the model and when a desired convergence is achieved, the model generated element volume is used to generate a corresponding simulated PW measurement that is stored in a library for future reference and use.

The library is populated with converged, calculated PW cycles that are generated using a range of model parameters including vascular parameters, such as vascular resistance, heart parameters, such as stroke volume and systolic fraction, and overall system parameters such as blood density and heart rate.

To generate a combinatorial library of synthetic PW cycles, one may begin with a first PW cycle using a starting value for each parameter. In a preferred embodiment, a first PW cycle is synthesized using a population average for each parameter and subsequent synthetic PW cycles are generated using multipliers, or scalars, with each parameter in many combinations. This technical feature provides the advantage of being able to update only one set of parameters to generate a completely new library. This can be useful as the accumulation of large amounts of clinical data allow improvements in, and provide greater understanding of, average parameter values for the general population as well as subpopulations based on factors such as age, gender, health status, vital signs, body surface area, body mass index, weight, height, and estimated levels of arterial calcification. Any number of sources may be used for population averages to be used. Values may be drawn, for example, from medical literature, a collection of measured clinical values, or both.

Bounds or limits may be set for one or more model parameters and these limits may be different for different subpopulations depending on age, gender, weight, height, systolic pressure, diastolic pressure, heart rate, etc. or combinations of these such as age/(age + diastolic pressure), (systolic pressure - diastolic pressure), (systolic pressure + diastolic pressure)/2, or diastolic pressure/total blood volume, etc. The scalars used may be, for example, 0.1, 0.2, 0.3, 0.4, 0.5, 0.6. 0.7, 0.8, 0.9, 1.1, 1.2, 1.3, 1.4, 1.5, 1.6, 1.7, 1.8, 1.9, 2, 2.5, 3, 3.5, 4, 5, 6, 7, 8, 9, or 10.

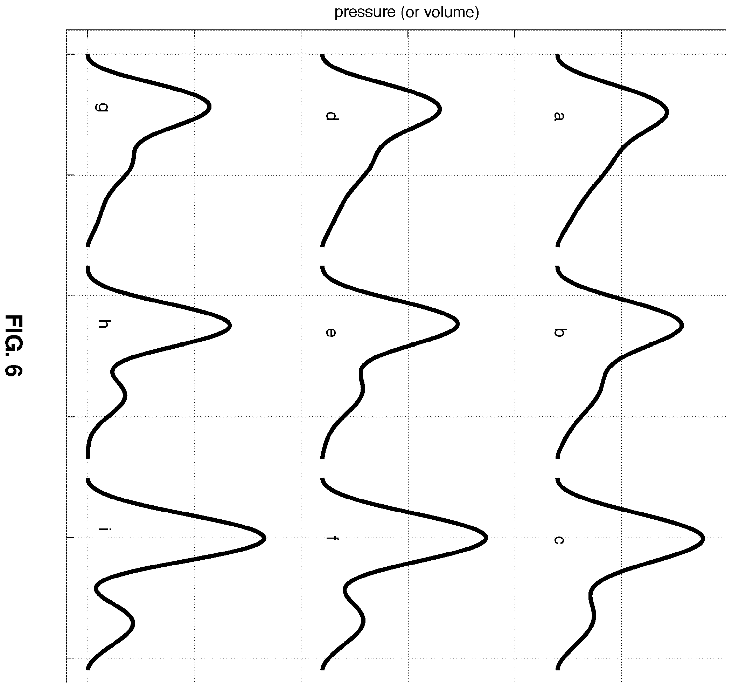

**Fig. 6** shows nine different synthetic PW cycles (a-i) generated using a seven segment human cardiovascular system model comprising resistance, compliance, and inertance as model parameters for each segment, and three model parameters for the heart, including systolic fraction, HR, and SV. Moving from the top row to the bottom row, aortic, arterial, arteriolar, and capillary resistance are each equal to baseline multiplied by a scalar of 1.4, 1.0, and 0.7, respectively for all segments. Moving from the first column to the third column from left to right, aortic, arterial, arteriolar, and capillary inertance are each equal to a baseline multiplied by a scalar of 1.0, 1.6, and 2.0, respectively for all model segments. Other parameters are set at baseline.

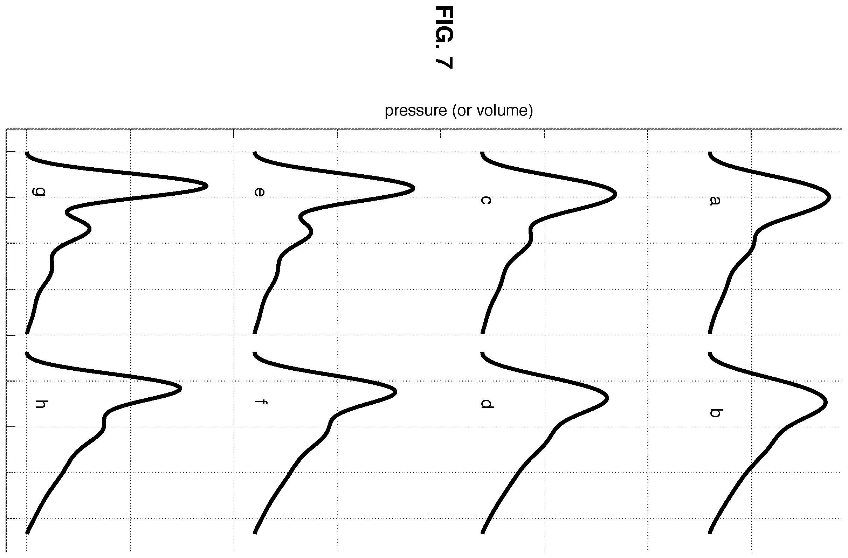

**Fig. 7** shows eight different synthetic PW cycles (a-h) generated using a seven segment human cardiovascular system model comprising resistance, compliance, and inertance as model parameters for each segment, and three model parameters for the heart, including systolic fraction, HR, and SV. Moving from the top row to the bottom row, systolic fraction is set to 0.34, 0.30, 0.24, and 0.20, respectively for all model segments. In the left column, resistance is set to a baseline value multiplied by a scalar of 0.7 for all segments. In the right column resistance is set to a baseline value multiplied by a scalar of 1.4 for all segments. **Figs. 6 and 7** show that changing parameters values results in synthetic PWs having different shapes. Ranges of scalars for each parameter may be limited by selected scalars to include physiologically possible states and avoid physiologically impossible states. The number of scalars applied to any or all of the parameters may be used to increase the number of PWs in a resulting PW library and/or the resolution of a resulting PW library.

The use of scalars as described herein is capable of generating many millions of synthetic PW cycles with unique combinations of model parameter values. Members of the PW library may initially be in the form of pressure vs. time and normalized to pressure vs. fraction of cycle or they may be generated as pressure vs. fraction of cycle directly. Plethysmographic data may be collected by direct measurement of pressure, for example by a pressure cuff or indirectly using a photoplethysmograph that calculates pressure changes from photoplethysmographic data. Additionally or alternatively, PWs may be generated as absorbance or transmittance vs. time or fraction of cycle. The latter may be useful for comparison with photoplethysmographic data collected from a patient in which absorbance is not converted to pressure. It is also possible to measure changes in blood and/or vessel diameter or volume using a stretching sensor or an impedance measurement. For such a case, PWs may be generated in the form of volume vs. time and/or fraction of cycle.

Comparing qualified query PW cycles to synthetic PW cycles stored on a computing device to identify one or more closest fits is much faster than previous methods that receive measured PW cycles as input and then use a computational model to estimate cardiovascular parameters such as CO and SV. This improved speed enables the noninvasive monitoring of CO and other cardiovascular parameters such as systolic fraction, resistance, aortic pressure, and pulmonary pressure at home and in clinical settings with monitored values being available within minutes of collecting PW data from a subject. The time required for measuring these cardiovascular parameters may be reduced further by reducing the number of library members screened to those possessing a limited range of values for age, weight, HR, body mass index, gender, and/or other physiological and/or demographic parameters.

A system (800) for measuring a cardiovascular parameter may comprise a monitoring or measuring device (801) that measures a time varying cardiovascular parameter and a computer (809) comprising a stored PW library (807) and executable code for receiving (101) and processing incoming data from the monitoring device (801) into the form necessary for screening (107) the PW library for a closest match and for reporting (108) the result of the screening (**Fig. 8**). The executable code may be stored on a non-transitory computer-readable storage medium integral to, or connected to, the computer (809). The measuring or monitoring device (801) may provide data directly to the data collection module or indirectly via a storage medium on which measured data is stored. The synthetic waveform library (807) may be stored on a non-transitory computer-readable storage medium integral to, or connected to, the computer (809). The system (800) may optionally comprise a non-transitory computer-readable storage medium comprising executable Library Generator (806) code for generating a synthetic waveform library.

Examples of cardiovascular parameters that may be measured include those cardiovascular parameters included in the HCS model and parameters that are readily calculated from these parameters. These parameters include stroke volume (SV), cardiac output (CO), elastance, compliance, resistance, inertance, aortic pressure, pulmonary pressure, venous pressure, cardiac power output, systolic fraction, and regurgitation fraction.

The monitoring/measuring device (801) is a device that measures a time varying cardiovascular parameter that is, or can be modified to be, in the form of a series or train of PWs. Examples of monitoring device (801) include a pulse oximeter, a pressure cuff adapted to measure time varying pressure of an artery or other blood vessel, an electrical impedance device measuring impedance in a part of the body to estimate changes in blood volume over time, a limb or whole body plethysmograph measuring changes in blood volume over time, and a sonograph measuring time varying blood flow in the heart or a blood vessel. The computing device (809) may be a tablet, laptop or desktop computer, smartphone, or a functional equivalent. The monitoring device and computing device are preferably configured such that measured time varying data is transmitted to the computing device and, optionally, a user interface of the computing device may be used to control the monitoring device. The computing device (809) and/ or a connected solid state or flash storage device may contain the synthetic PW cycle library (807) as well as software modules for performing the methods described.

## Claims

1. A computer implemented method for noninvasively measuring a value of cardiovascular parameter of a subject, said method comprising:

providing a library (807) of synthetic PW cycles;

splitting (103) a plurality of time varying pulse plethysmographic or pulse pressure waveform (PW) cycles into individual PW cycles by identifying start and end points for said plurality of PW cycles;

applying a quality metric (104) to select an individual PW cycle as a query cycle;

screening (107) said library of synthetic PW cycles with the query cycle using a difference metric calculation to identify at least one synthetic PW cycle as a solution PW cycle that best fits a shape of the query cycle; and

reporting (108) one or more model parameters associated with said solution PW cycle as the value of the measured cardiovascular parameter

wherein said library of synthetic PW cycles comprises a plurality of unique computer simulated PW cycles with each computer simulated PW cycle being associated with a unique combination of model parameters of a computational cardiovascular model used to generate said library of synthetic PW cycles.

2. The method of claim 1, wherein said cardiovascular parameter is selected from stroke volume, systolic fraction, inertance, resistance, aortic pressure, central venous pressure, pulmonary pressure, compliance, regurgitation fraction, and combinations thereof.

3. The method of either of claims 1 or 2, further comprising executing a library generator (806) code to generate said library of synthetic PW cycles using said a computational cardiovascular model.

4. The method of any of claims 1-3, wherein said computational cardiovascular model comprises a segmented computational fluid dynamic model of a cardiovascular system or a lumped parameter model.

5. The method of any of claims 1-4, wherein said difference metric calculation comprises a multiplied-dimension distance or similarity function kernel operating on the one or more query PW cycles and the synthetic PW cycles to achieve a consensus effect that identifies the solution synthetic PW cycle.

6. The method of any of claims 1-5, wherein said screening comprises averaging of estimated parameters for K Nearest Neighbor (KNN) queries and adjusting a K factor in the KNN query.

7. The method of any of claims 1-6, wherein said PW data is photoplethysmogram pulse pressure data.

8. The method of claim 3, wherein said executing a library generator (806) code comprises multiplying baseline values of cardiovascular model parameters by scalar values to generate PWs having different shapes.

9. The method of claim 8, wherein said executing a library generator (806) code results in at least millions of synthetic PW cycles with unique combinations of model parameter values.

10. The method of any of claims 1-9, comprising:

applying a quality metric (104) to select a plurality of individual cycles as a plurality of query cycles and screening (107) the library of synthetic PW cycles with the plurality of query cycles

using a difference metric calculation to identify at least one synthetic PW cycle as a solution PW cycle that best fits shapes of the plurality of query cycles.

11. The method of any of claims 1-9, comprising:

normalizing (105) said individual cycle over a cycle duration to generate a normalized query cycle and screening (107) said library of synthetic PW cycles with the normalized query cycle using a difference metric calculation to identify at least one synthetic PW cycle as a solution PW cycle that best fits a shape of the normalized query cycle; and

wherein the normalized query cycle has the same resolution and is in phase with the library of synthetic PW cycles.

12. The method of claim 11, wherein a plurality of individual cycles are selected and normalized to produce a plurality of normalized query cycles combined into a continuous series of normalized query cycles.

13. A system (800) for monitoring a cardiovascular parameter, said system comprising a pulse oximeter in communication with a computing device comprising a user interface wherein:
the computing device comprises software configured to receive plethysmographic waveform data from the pulse oximeter, a stored library of synthetic PW cycles, and executable software configured to perform the method of claim 7.

14. The system of claim 13, wherein the pulse oximeter and the computing device are configured for the pulse oximeter to be controlled by user input entered into the user interface of the computing device.

15. A non-transitory computer-readable storage medium storing a program that causes a computer to execute a method for noninvasively measuring a value of cardiovascular parameter of a subject, said method comprising:

receiving (101) a data set comprising time varying pulse pressure-wave (PW) data or time varying pulse volume-wave data for the subject as input, said dataset comprising a plurality of cycles;
identifying start and end points for the plurality of cycles;
selecting one or more cycles to produce one or more query PW cycles;
screening a library (807) of synthetic PW cycles with the one or more query PW cycles using a difference metric calculation to identify at least one solution synthetic PW cycle that best fits a shape of the one or more query PW cycles; and
reporting (108) one or more model parameters associated with said at least one solution synthetic PW cycle as the measured value of cardiovascular parameter;
wherein:

said library of synthetic PW cycles comprises a plurality of unique computer simulated PW cycles with each computer simulated PW cycle being associated with a unique combination of model parameters of a computational cardiovascular model used to generate said library of synthetic PW cycles and
said computational cardiovascular model comprises stroke volume and heart rate as model parameters.

**Patentansprüche**

1. Computerimplementiertes Verfahren zum nichtinvasiven Messen eines Werts eines kardiovaskulären Parameters eines Subjekts, wobei das Verfahren umfasst:

Bereitstellen einer Bibliothek (807) von synthetischen PW-Zyklen;
Aufteilen (103) einer Vielzahl von zeitlich variierenden pulsplethysmographischen oder Pulsdruckwellenform(PW)-Zyklen in einzelne PW-Zyklen durch Identifizieren von Start- und Endpunkten für die Vielzahl von PW-Zyklen;
Anwenden einer Qualitätsmetrik (104), um einen einzelnen PW-Zyklus als einen Abfragezyklus auszuwählen;
Screening (107) der Bibliothek von synthetischen PW-Zyklen mit dem Abfragezyklus unter Verwendung einer Differenzmetrikberechnung, um mindestens einen synthetischen PW-Zyklus als einen Lösungs-PW-Zyklus zu identifizieren, der am besten zu einer Form des Abfragezyklus passt; und
Ausgeben (108) eines oder mehrerer Modellparameter, die mit dem Lösungs-PW-Zyklus verbunden sind, als den Wert des gemessenen kardiovaskulären Parameters,
wobei die Bibliothek von synthetischen PW-Zyklen eine Vielzahl von einmaligen computersimulierten PW-Zyklen umfasst, wobei jeder computersimulierte PW-Zyklus mit einer einmaligen Kombination von Modellparametern eines computergestützten kardiovaskulären Modells verbunden ist, das zum Erzeugen der Bibliothek von synthetischen PW-Zyklen verwendet wird.

2. Verfahren nach Anspruch 1, wobei der kardiovaskuläre Parameter ausgewählt ist aus Schlagvolumen, systolischer Fraktion, Inertanz, Widerstand, Aortendruck, zentralvenösem Druck, Lungendruck, Compliance, Regurgitationsfraktion und Kombinationen davon.

3. Verfahren nach einem der Ansprüche 1 oder 2, ferner umfassend Ausführen eines Bibliothekserzeugungscodes (806), um die Bibliothek von synthetischen PW-Zyklen unter Verwendung des computergestützten kardiovaskulären Modells zu erzeugen.

4. Verfahren nach einem der Ansprüche 1-3, wobei das computergestützte kardiovaskuläre Modell ein segmentiertes computergestütztes fluiddynamisches Modell eines kardiovaskulären Systems oder ein Modell mit zusammengefassten Parametern umfasst.

**5.** Verfahren nach einem der Ansprüche 1-4, wobei die Differenzmetrikberechnung einen Abstandsmultiplikations- oder Ähnlichkeitsfunktionskern umfasst, der an dem einen oder den mehreren Abfrage-PW-Zyklen und den synthetischen PW-Zyklen arbeitet, um einen Konsenseffekt zu erzielen, der den synthetischen Lösungs-PW-Zyklus identifiziert.

**6.** Verfahren nach einem der Ansprüche 1-5, wobei das Screening Mittelwertbildung von geschätzten Parametern für K Nächster-Nachbar(KNN)-Abfragen und Anpassen eines K-Faktors in der KNN-Abfrage umfasst.

**7.** Verfahren nach einem der Ansprüche 1-6, wobei die PW-Daten Photoplethysmogramm-Pulsdruckdaten sind.

**8.** Verfahren nach Anspruch 3, wobei das Ausführen eines Bibliothekserzeugungs(806)-Codes Multiplizieren von Grundlinienwerten von kardiovaskulären Modellparametern mit skalaren Werten umfasst, um PWs mit unterschiedlichen Formen zu erzeugen.

**9.** Verfahren nach Anspruch 8, wobei das Ausführen eines Bibliothekserzeugungs(806)-Codes mindestens Millionen von synthetischen PW-Zyklen mit einmaligen Kombinationen von Modellparameterwerten liefert.

**10.** Verfahren nach einem der Ansprüche 1-9, umfassend:

Anwenden einer Qualitätsmetrik (104) zum Auswählen einer Vielzahl von einzelnen Zyklen als eine Vielzahl von Abfragezyklen und
Screening (107) der Bibliothek von synthetischen PW-Zyklen mit der Vielzahl von Abfragezyklen unter Verwendung einer Differenzmetrikberechnung, um mindestens einen synthetischen PW-Zyklus als einen Lösungs-PW-Zyklus zu identifizieren, der am besten zu Formen der Vielzahl von Abfragezyklen passt.

**11.** Verfahren nach einem der Ansprüche 1-9, umfassend:

Normieren (105) des individuellen Zyklus über eine Zyklusdauer, um einen normierten Abfragezyklus und zu erzeugen
Screening (107) der Bibliothek von synthetischen PW-Zyklen mit dem normierten Abfragezyklus unter Verwendung einer Differenzmetrikberechnung, um mindestens einen synthetischen PW-Zyklus als einen Lösungs-PW-Zyklus zu identifizieren, der am besten zu einer Form des normierten Abfragezyklus passt; und
wobei der normierte Abfragezyklus die gleiche Auflösung aufweist und in Phase mit der Bibliothek von synthetischen PW-Zyklen ist.

**12.** Verfahren nach Anspruch 11, wobei eine Vielzahl von einzelne Zyklen ausgewählt und normiert wird, um eine Vielzahl von normierten Abfragezyklen zu erzeugen, die zu einer kontinuierlichen Reihe von normierten Abfragezyklen kombiniert sind.

**13.** System (800) zum Überwachen eines kardiovaskulären Parameters, wobei das System ein Pulsoximeter in Kommunikation mit einer Rechenvorrichtung umfasst, die eine Benutzerschnittstelle umfasst, wobei:
die Rechenvorrichtung Software, die dazu ausgelegt ist, plethysmographische Wellenformdaten von dem Pulsoxymeter zu erhalten, eine gespeicherte Bibliothek von synthetischen PW-Zyklen und ausführbare Software, die dazu ausgelegt ist, das Verfahren nach Anspruch 7 durchzuführen, umfasst.

**14.** System nach Anspruch 13, wobei das Pulsoxymeter und die Rechenvorrichtung dazu ausgelegt sind, das Pulsoxymeter durch Benutzereingabe, die in die Benutzerschnittstelle der Rechenvorrichtung eingegeben wird, zu steuern.

**15.** Nichtflüchtiges computerlesbares Speichermedium, das ein Programm speichert, das einen Computer veranlasst, ein Verfahren zum nichtinvasiven Messen eines Werts eines kardiovaskulären Parameters eines Subjekts auszuführen, wobei das Verfahren umfasst:

Empfangen (101) eines Datensatzes, der zeitlich variierende Pulsdruckwellen(PW)-Daten oder zeitlich variierende Pulsvolumenwellen-Daten für das Subjekt umfasst, als Eingabe, wobei der Datensatz eine Vielzahl von Zyklen umfasst;
Identifizieren von Start- und Endpunkten für die Vielzahl von Zyklen;
Auswählen eines oder mehrerer Zyklen, um einen oder mehrere Abfrage-PW-Zyklen zu erzeugen;
Screening einer Bibliothek (807) von synthetischen PW-Zyklen mit dem einen oder den mehreren Abfrage-PW-Zyklen unter Verwendung einer Differenzmetrikberechnung, um mindestens einen synthetischen Lösungs-PW-Zyklus zu identifizieren, der am besten zu einer Form des einen oder der mehreren Abfrage-PW-Zyklen passt; und

Ausgeben (108) eines oder mehrerer Modellparameter, die mit dem mindestens einen synthetischen Lösungs-PW-Zyklus verbunden sind, als den gemessenen Wert des kardiovaskulären Parameters; wobei:

die Bibliothek von synthetischen PW-Zyklen eine Vielzahl von einmaligen computersimulierten PW-Zyklen umfasst, wobei jeder computersimulierte PW-Zyklus mit einer einmaligen Kombination von Modellparametern eines computergestützten kardiovaskulären Modells verbunden ist, das zum Erzeugen der Bibliothek von synthetischen PW-Zyklen verwendet wird, und

das computergestützte kardiovaskuläre Modell Schlagvolumen und Herzfrequenz als Modellparameter umfasst.

## Revendications

1. Procédé mis en œuvre par ordinateur permettant de mesurer de manière non invasive une valeur de paramètre cardiovasculaire d'un sujet, ledit procédé comprenant :

la fourniture d'une bibliothèque (807) de cycles PW synthétiques ;

la division (103) d'une pluralité de cycles de forme d'onde de pléthysmographie à impulsions ou de pression (PW) à impulsions variant dans le temps en cycles PW individuels par identification de points de début et de fin pour ladite pluralité de cycles PW ;

l'application d'une métrique de qualité (104) pour sélectionner un cycle PW individuel en tant que cycle d'interrogation ;

le tri (107) de ladite bibliothèque de cycles PW synthétiques avec le cycle d'interrogation à l'aide d'un calcul de métrique de différence pour identifier au moins un cycle PW synthétique en tant que cycle PW de solution qui s'adapte le mieux à une forme du cycle d'interrogation ; et

le rapport (108) d'un ou de plusieurs paramètres de modèle associés audit cycle PW de solution en tant que valeur du paramètre cardiovasculaire mesuré

dans lequel ladite bibliothèque de cycles PW synthétiques comprend une pluralité de cycles PW simulés par ordinateur uniques, chaque cycle PW simulé par ordinateur étant associé à une combinaison unique de paramètres de modèle d'un modèle cardiovasculaire informatique utilisé pour générer ladite bibliothèque de cycles PW synthétiques.

2. Procédé selon la revendication 1, dans lequel ledit paramètre cardiovasculaire est choisi parmi volume systolique, fraction systolique, inertance, résistance, pression aortique, pression veineuse centrale, pression pulmonaire, compliance, fraction de régurgitation et des combinaisons de ceux-ci.

3. Procédé selon l'une ou l'autre des revendications 1 et 2, comprenant en outre l'exécution d'un code de générateur de bibliothèque (806) pour générer ladite bibliothèque de cycles PW synthétiques en utilisant ledit modèle cardiovasculaire informatique.

4. Procédé selon l'une quelconque des revendications 1 à 3, dans lequel ledit modèle cardiovasculaire informatique comprend un modèle dynamique informatique segmenté de fluide d'un système cardiovasculaire ou un modèle de paramètres groupés.

5. Procédé selon l'une quelconque des revendications 1 à 4, dans lequel ledit calcul de métrique de différence comprend un noyau de fonction de distance ou de similarité à dimension multipliée fonctionnant sur le ou les cycles PW d'interrogation et les cycles PW synthétiques pour obtenir un effet de consensus qui identifie le cycle PW synthétique de solution.

6. Procédé selon l'une quelconque des revendications 1 à 5, dans lequel ledit tri comprend le calcul de la moyenne de paramètres estimés pour des interrogations des K plus proches voisins(KNN) et l'ajustement d'un facteur K dans l'interrogation KNN.

7. Procédé selon l'une quelconque des revendications 1 à 6, dans lequel lesdites données PW sont des données de pression à impulsions de photopléthysmogramme.

8.  Procédé selon la revendication 3, dans lequel ladite exécution d'un code de générateur de bibliothèque (806) comprend la multiplication de valeurs de base de paramètres de modèle cardiovasculaire par des valeurs scalaires pour générer des PW ayant des formes différentes.

9.  Procédé selon la revendication 8, dans lequel ladite exécution d'un code de générateur de bibliothèque (806) a pour résultat au moins des millions de cycles PW synthétiques avec des combinaisons uniques de valeurs de paramètres de modèle.

10. Procédé selon l'une quelconque des revendications 1 à 9, comprenant :

    l'application d'une métrique de qualité (104) pour sélectionner une pluralité de cycles individuels en tant que pluralité de cycles d'interrogation et
    le tri (107) de la bibliothèque de cycles PW synthétiques avec la pluralité de cycles d'interrogation à l'aide d'un calcul de métrique de différence pour identifier au moins un cycle PW synthétique en tant que cycle PW de solution qui s'adapte le mieux à des formes de la pluralité de cycles d'interrogation.

11. Procédé selon l'une quelconque des revendications 1 à 9, comprenant :

    la normalisation (105) dudit cycle individuel sur une durée de cycle pour générer un cycle d'interrogation normalisé et
    le tri (107) de ladite bibliothèque de cycles PW synthétiques avec le cycle d'interrogation normalisé à l'aide d'un calcul de métrique de différence pour identifier au moins un cycle PW synthétique en tant que cycle PW de solution qui s'adapte le mieux à une forme du cycle d'interrogation normalisé ; et
    dans lequel le cycle d'interrogation normalisé a la même résolution et est en phase avec la bibliothèque de cycles PW synthétiques.

12. Procédé selon la revendication 11, dans lequel une pluralité de cycles individuels est sélectionnée et normalisée pour produire une pluralité de cycles d'interrogation normalisés combinés en une série continue de cycles d'interrogation normalisés.

13. Système (800) de surveillance d'un paramètre cardiovasculaire, ledit système comprenant un oxymètre de pouls en communication avec un dispositif informatique comprenant une interface utilisateur dans lequel :
    le dispositif informatique comprend un logiciel configuré pour recevoir des données de forme d'onde pléthysmographique provenant de l'oxymètre de pouls, une bibliothèque stockée de cycles PW synthétiques, et un logiciel exécutable configuré pour réaliser le procédé selon la revendication 7.

14. Système selon la revendication 13, dans lequel l'oxymètre de pouls et le dispositif informatique sont configurés pour que l'oxymètre de pouls soit commandé par une entrée utilisateur saisie dans l'interface utilisateur du dispositif informatique.

15. Support de stockage non transitoire lisible par ordinateur stockant un programme qui amène un ordinateur à exécuter un procédé de mesure de manière non invasive d'une valeur de paramètre cardiovasculaire d'un sujet, ledit procédé comprenant :

    la réception (101) d'un ensemble de données comprenant des données d'onde de pression (PW) à impulsions variant dans le temps ou des données d'onde de volume à impulsions variant dans le temps pour le sujet en tant qu'entrée, ledit ensemble de données comprenant une pluralité de cycles ;
    l'identification de points de début et de fin pour la pluralité de cycles ;
    la sélection d'un ou de plusieurs cycles pour produire un ou plusieurs cycles PW d'interrogation ;
    le tri d'une bibliothèque (807) de cycles PW synthétiques avec le ou les cycles PW d'interrogation à l'aide d'un calcul de métrique de différence pour identifier au moins un cycle PW synthétique de solution qui s'adapte le mieux à une forme du ou des cycles PW d'interrogation ; et
    le rapport (108) d'un ou de plusieurs paramètres de modèle associés audit au moins un cycle PW synthétique de solution en tant que valeur mesurée du paramètre cardiovasculaire ;
    dans lequel
    ladite bibliothèque de cycles PW synthétiques comprend une pluralité de cycles PW simulés par ordinateur uniques, chaque cycle PW simulé par ordinateur étant associé à une combinaison unique de paramètres de modèle d'un modèle cardiovasculaire informatique utilisé pour générer ladite bibliothèque de cycles PW synthétiques et ledit modèle cardiovasculaire informatique comprend le volume systolique et la fréquence cardiaque en tant que paramètres du modèle.

**Fig. 1**

Fig. 2

FIG. 3

EP 3 989 808 B1

EP 3 989 808 B1

Measured PW

**A**

Normalized Qualified PW

HR=75 BPM

**B**

**FIG. 4**

20

20

FIG. 5

**FIG. 6**

**FIG. 7**

800

Measuring Device
801

Data Collection Module
802

Computer
809

Quality Metric Module
803

Library Generator
806

Screening Module
804

Library
807

Medical Device
808

Display / Report
805

**FIG. 8**

**EP 3 989 808 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010124034 A
- US 8494829 B1
- US 9060722 B1
- US 9173574 B1
- US 9375171 B
- US 9451886 B1
- US 9649036 B1
- US 20170119261 A1
- EP 2512325 B1
- WO 2017220353 A